# EUROPEAN PATENT APPLICATION

(11) **EP 4 091 592 A1**
(43) Date of publication of application: **23.11.2022**
(21) Application number: 22174099.6
(22) Date of filing: 18.05.2022
(51) Int. Cl.: A61F 13/15

(54) **ABSORBENT AND WASHABLE PANTY FOR DIFFERENT LEVELS OF MENSTRUAL AND URINARY LOSSES**

(30) Priority: 20.05.2021 IT 202100013097
(71) Applicant: Steinmann & Co. S.a.S. Di Colombo Cristina, 20857 Camparada, Monza-Brianza (IT)
(72) Inventor: CORONA STEINMANN, Valentina, I-20857 Camparada, MONZA E BRIANZA (IT)
(74) Representative: Gualeni, Nadia

(57) **Abstract**

A panty (1) according to the present invention comprises a panty body (11) provided with an inguinal portion (13) having:
- a top layer (21) to at least partially absorb said losses;
- a layer (23) that is impermeable to said losses;
- a pocket (22) defined between the upper layer (21) and the impermeable layer (23), into which an absorbent and removable insert (3) may be at least partially inserted through an opening (24). Advantageously, the present panty may optimally absorb flow losses, either through the inguinal portion or through the removable insert, adapting to different menstrual or urine flows.

## Description

The present invention is generally in the field of absorbent panties for menstrual losses or moderate urinary losses.

Disposable absorbent panties composed of three layers are known in the sector: a first draining layer in contact with the skin, a second flow-absorbing state, and a third impermeable layer.

An initial disadvantage of said disposable absorbent panties lies precisely in the fact that they are disposable and therefore generate a great deal of waste. An additional disadvantage lies in the fact that such panties are only able to absorb a certain amount of flow, which is precisely why it is necessary to have different formats of panties depending on the flow to be absorbed.

Also known in the sector are washable absorbent panties, which, although more environmentally friendly, do not solve the problem of having to have different panty formats depending on the flow to be absorbed. Further, said known panties are difficult to wash thoroughly due to the presence of the three layers-draining, absorbent, impermeable-which are sewn together.

The object of the present invention is to provide a washable absorbent panty that is able to absorb different menstrual or urine flows.

Further, it is the object of the present invention to provide a washable absorbent panty that is thoroughly washable.

This is achieved by a panty according to claim 1, as well as by a panty kit according to claim 7. The dependent claims describe preferred variant embodiments of the invention.

The features and the advantages of the present invention will appear more clearly from the following description, made by way of an indicative and nonlimiting example with reference to the accompanying figures, in which:
- Fig. 1A and 1B show a front view and a rear view, respectively, of a washable absorbent panty according to the present invention;
- Fig. 2 is a view of the inner inguinal portion of the panty of Fig. 1, in a variant embodiment;
- Fig. 3 is an exploded view of the layers that make up the inner inguinal portion of the panty of Fig. 2;

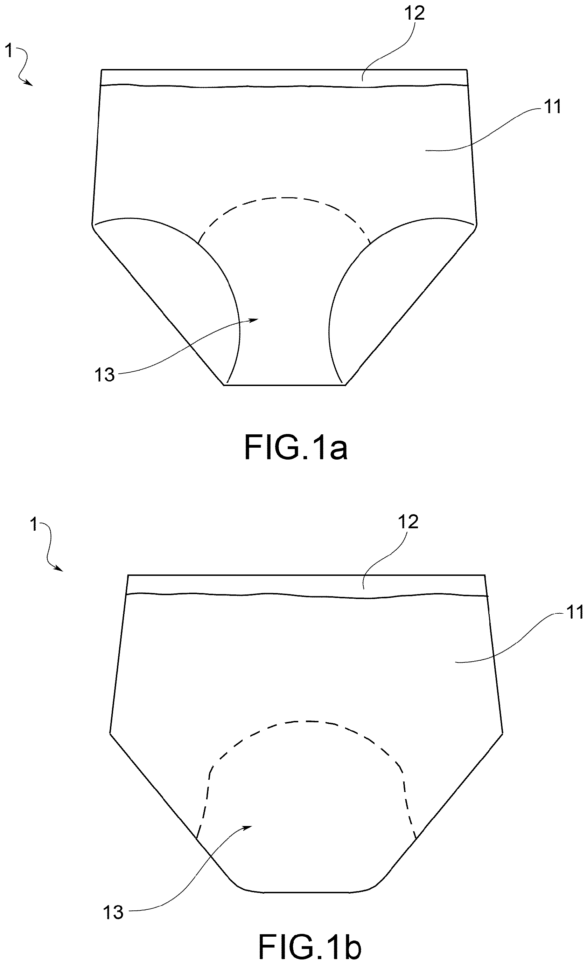

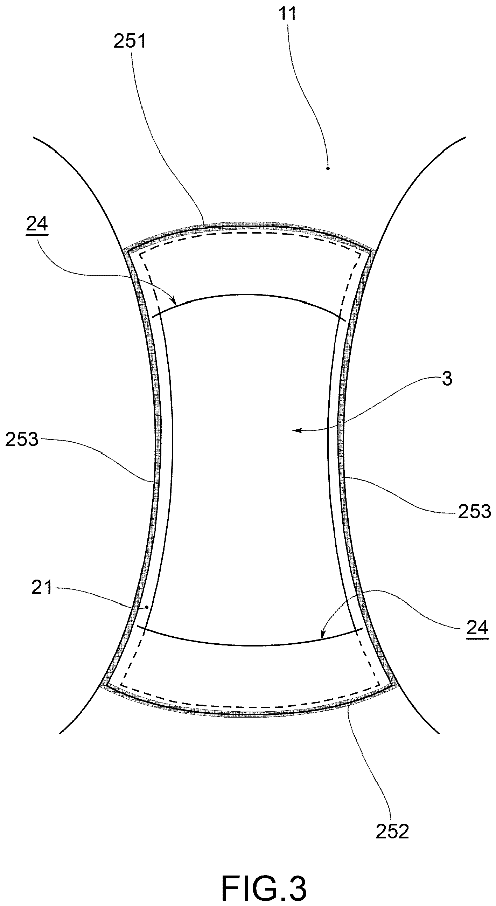

- Fig. 3 is a view of the inner inguinal portion of the panty of Fig. 1, in a further variant embodiment;

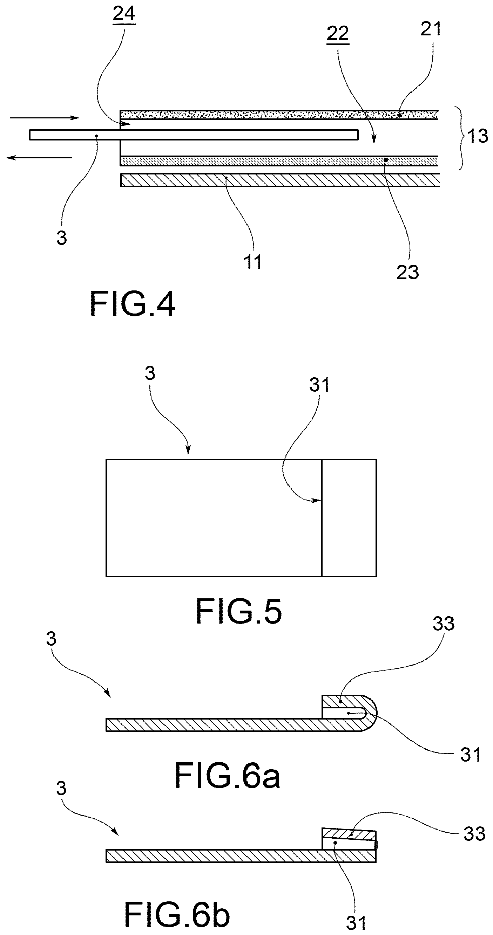

- Fig. 5 is a front view of a component of the panty of Fig. 1, in particular of an absorbent insert;
- Fig. 6A and 6B are cross-sectional views of two variant embodiments of the absorbent insert in Fig. 1.

With reference to the appended figures, a washable absorbent panty according to the present invention has been indicated with reference number 1.

The panty 1, shown in its front view in Fig. 1A and its rear view in Fig. 1B, comprises a panty body 11, preferably provided with a waistband 12, and an inguinal portion 13.

The panty body 11 is made of fabric, preferably stretch, such as cotton, jersey, or tencel.

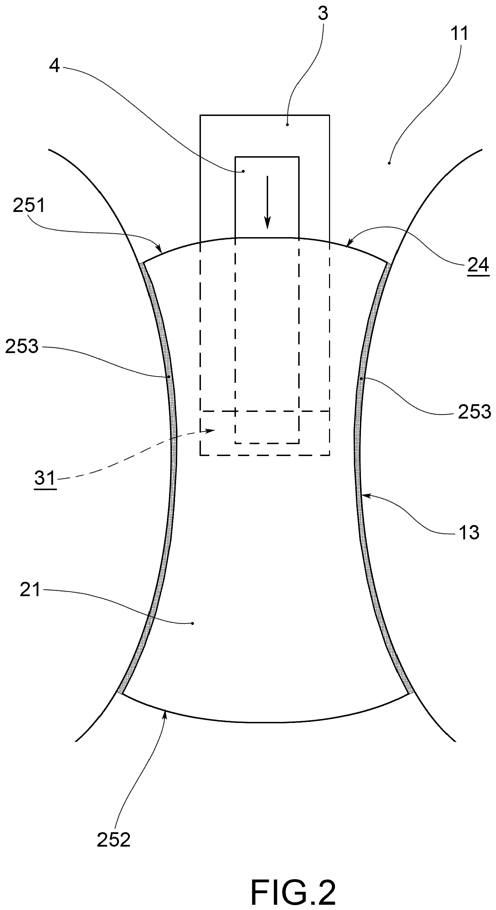

The inguinal portion 13, shown in Fig. 2 and 3, is defined by a front edge 251, a rear edge 252, and opposing lateral edges 253.

The inguinal portion 13 consists of:
- an upper layer 21 made of fabric, intended to be in contact with the skin and to at least partially absorb flow losses;
- an impermeable layer 23, intended to prevent losses of flows on the panty body 11.

In fact, as may be seen in Fig. 4, the panty body 11 extends below the impermeable layer 23 so that the inguinal portion 13 is invisible from the outside.

The upper layer 21 and the impermeable layer 23 of the inguinal portion 13 are attached to the panty body 11 at least at the side edges 253, such as by seams, preferably also at the rear edge 252.

A pocket 22 is defined between the upper layer 21 and the impermeable layer 23 of the inguinal portion 13 suitable to accommodate at least partially an absorbent and removable insert 3.

The pocket 22 has at least one opening 24 for the insertion and extraction of the insert 3.

Preferably, the pocket 22 has two openings 24, one in front and one in back, for the insertion and extraction of the insert 3.

In an example embodiment, shown in Fig. 2, the insert 3 is fully inserted inside the pocket 22.

In a first variant of this example, the pocket 22 has an opening 24 in front for insertion and extraction of the insert 3. Preferably, the opening 24 of the pocket 22 coincides with the front edge 251 of the inguinal portion 13.

In a second variant of this example, the pocket 22 has a rear opening 24 for the insertion and extraction of the insert 3. Preferably, the opening 24 of the pocket 22 coincides with the rear edge 252 of the inguinal portion 13.

In a third variant of this example, the pocket 22 has both a front opening 24 and a rear opening 24 for insertion and extraction of the insert 3.

In a fourth variant of this example, the pocket 22 has a central opening 24 for the insertion and extraction of the insert 3.

In a further example embodiment, shown in Fig. 3, the insert 3 is partially insertable inside the pocket 22. That is, only the front and rear ends of the insert 3 are inserted inside the pocket 22. Preferably, the pocket 22 has both a front opening 24 and a rear opening 24, each made through the upper layer (21). Preferably, both the front opening 24 and the rear opening 24 of the pocket 22 do not coincide with the respective front edge 251 and rear edge 252, but are made upstream thereof. In said example, the upper layer 21 and the impermeable layer 23 of the inguinal portion 13 are attached to the panty body 11 at all the edges (side 253, rear 252, and front 251), e.g., by seams.

The absorbent and removable insert 3, shown in Fig. 5, is made of natural or synthetic absorbent material.

The insert 3 may be disposable or washable.

The insert 3 may be single-layer or multi-layer.

Preferably, the insert 3 is made in different variants of material and thicknesses depending on the desired level of absorbency, so that a plurality of inserts 3 is available depending on the flow to be absorbed.

To facilitate the insertion of the insert 3 into the pocket 22 of the inguinal portion 13, a thrust means 4, such as a wooden blade, may be used.

To facilitate pushing, the insert 3 comprises a grip portion 31 in which the thrust means 4 is engageable.

Preferably, the grip portion 31 is a pocket, obtained, for example, by folding one end 32 of the insert 3 back on itself, as in Fig. 6A, or by applying a gusset 33 to the insert 3, as in Fig. 6B.

In an example embodiment, the grip portion 31 occupies only a portion of the end of the insert 3. In a further example embodiment, the grip portion 31 extends the entire length of the insert 3. In this example, the grip portion is obtained by sewing two layers of fabric on three sides, or by refolding in half and sewing a single strip of fabric on three sides.

Also the subject of the invention is a panty kit comprising:
- a panty 1 with an inguinal portion 13 equipped with a pocket 22;
- at least one absorbent and removable insert 3 that may be inserted at least partially into the pocket 22 of the panty 1.

Preferably, the panty kit comprises at least two inserts 3 with different levels of absorbency.

Preferably, the panty kit also comprises a thrust means 4 of the insert 3, such as a blade, which is insertable into the grip portion 31 of said insert 3.

In an example embodiment, the panty kit mentioned above is completely made of reusable materials.

The washable absorbent panty according to the present invention is preferably elasticized, made of comfortable and durable fabric, and provided with smooth and uniform seam lines that do not irritate.

The washable absorbent panty according to the present invention may also, but not necessarily, be used together with sanitary pads, both washable and disposable, both for maternity and incontinence, as well as with tampons, panty liners, and menstrual cups. In said use, the panty provides extra protection against the unexpected overflow from traditional absorbent systems.

The washable absorbent panty according to the present invention may also be used in place of a panty liner between menstrual cycles.

Innovatively, the washable absorbent panty according to the present invention is capable of absorbing different menstrual or urine flows.

Advantageously, the washable absorbent panty according to the present invention is able to optimally absorb flow losses, either by means of the inguinal portion or by means of the removable insert.

Advantageously, the washable absorbent panty according to the present invention, due to the removability of the insert, is easily washed thoroughly, even in a washing machine.

Advantageously, the present panty looks like underwear and offers discreet protection against losses.

It is clear that a person skilled in the art, in order to satisfy contingent and specific needs, could make modifications to the panty and to the kit as described above, said modifications being all contained within the scope of protection as defined in the following claims.

## Claims

1. An absorbent and washable panty (1) for menstrual and urinary losses, comprising a panty body (11) provided with an inguinal portion (13) having:
- an upper layer (21) to absorb said losses at least partially;
- a layer (23) impermeable to said losses;
- a pocket (22) defined between the upper layer (21) and the impermeable layer (23), into which an absorbent and removable insert (3) is at least partially insertable through an opening (24).

2. A panty (1) according to claim 1, wherein said opening (24) is a front opening or a rear opening made through the upper layer (21).

3. A panty (1) according to claim 1 or 2, wherein an absorbent and removable insert (3) is completely insertable into the pocket (22).

4. A panty (1) according to claim 3, wherein said opening (24) is a front opening and coincides with a front edge (251) of the inguinal portion (13) or is a rear opening and coincides with a rear edge (252) of the inguinal portion (13).

5. A panty (1) according to claim 2 or 4, wherein said pocket (22) comprises both a front opening (24) and a rear opening (24).

6. A panty (1) according to any one of the preceding claims, wherein the panty body (11) extends below the impermeable layer (23) so that the inguinal portion (13) is invisible from the outside.

7. An absorbent and washable panty kit for menstrual and urinary losses, comprising:
- a panty (1) according to any one of the preceding claims;
- at least one absorbent and removable insert (3) which is at least partially insertable into the pocket (22) of the panty (1).

8. A panty kit according to claim 7, wherein the insert (3) comprises a grip portion (31) for a thrust means (4), said grip portion (31) being a pocket obtained by folding the insert (3) on itself or by applying a gusset (33) to the insert (3).

9. A panty kit according to claim 7, further comprising a thrust means (4) of the insert (3) which is insertable into said grip portion (31) of the insert (3).

10. A panty kit according to any one of claims 7 to 9, comprising at least two inserts (3) with different levels of absorbency.
